# FASCICULE DE BREVET EUROPEEN

(11) **EP 2 039 390 B1**
(45) Date de publication et mention de la délivrance du brevet: **10.11.2010**
(21) Numéro de dépôt: 08290884.9
(22) Date de dépôt: 18.09.2008
(51) Int. Cl.: A61M 25/06

(54) **Outil de découpe de la gaine tubulaire d'un cathéter-guide en présence d'une sonde logée dans cette gaine**
Schneidewerkzeug für Hüllrohr eines Führungskatheters bei gleichzeitiger Anwesenheit einer Sonde in diesem Hüllrohr
Tool for cutting the tubular sheath of a catheter-guide in the presence of a probe lodged in this sheath

(30) Priorité: 18.09.2007 FR 0706550
(43) Date de publication de la demande: 25.03.2009
(73) Titulaire: Ela Medical, 92541 Montrouge (FR)
(72) Inventeur: Shan, Nicolas, 94300 Vincennes (FR); Ollivier, Jean-François, 91190 Villiers Le Bacle (FR)
(74) Mandataire: Dupuis-Latour, Dominique

(56) Documents cités:
- EP-A- 0 391 544
- US-A- 5 188 606
- US-A1- 2005 182 435
- US-A1- 2006 167 417
- US-A1- 2007 079 511
- US-A1- 2007 175 049
- US-B1- 6 497 681

## Description

L'invention concerne la mise en place des sondes intracorporelles, notamment les sondes de détection/stimulation cardiaque des "dispositifs médicaux implantables actifs" tels que définis par la directive 90/385/CEE du 20 juin 1990 du Conseil des communautés européennes, plus précisément des implants cardiaques de stimulation, de resynchronisation, de cardioversion et/ou de défibrillation.

Les sondes cardiaques peuvent être des sondes endocavitaires, comme les sondes de détection/stimulation des cavités droites, ou bien des sondes introduites dans le réseau coronarien, notamment les sondes comportant une électrode disposée face à une cavité gauche du myocarde.

L'introduction de ce dernier type de sonde, opérée par des voies endocavitaires, est une intervention particulièrement délicate compte tenu d'une part de la difficulté d'accès à l'entrée du sinus coronaire via l'oreillette droite, et d'autre part de la précision requise pour les points de stimulation une fois la sonde guidée et immobilisée dans le réseau coronarien.

L'une des techniques de mise en place d'une telle sonde met en oeuvre un accessoire dit "cathéter-guide". Cet accessoire comporte une gaine tubulaire creuse armée par un treillis métallique et dont la surface intérieure présente un faible coefficient de frottement (par exemple une surface de PTFE co-extrudée ou co-moulée avec le reste de la gaine). La flexibilité de la gaine est en outre étudiée de manière à présenter une rigidité en torsion suffisante pour permettre la transmission d'un mouvement de rotation d'une extrémité à l'autre, afin de pouvoir diriger l'extrémité de la sonde par rapport au myocarde lors de l'intervention.

Une fois posé, le cathéter-guide constitue un "tunnel" direct entre le "monde extérieur" et le sinus coronaire, tunnel que le chirurgien peut alors utiliser pour glisser la sonde jusqu'à son emplacement définitif.

Une fois la sonde installée, le cathéter-guide doit être extrait, et cette manoeuvre est délicate car il faut bien entendu éviter de déloger la sonde ou modifier son positionnement et son orientation.

Ces cathéters doivent être de diamètre minimal pour permettre leur navigation dans les vaisseaux sanguins en étant moins traumatiques et plus facilement guidables. De plus, les dimensions du cathéter sont ajustées à la sonde pour une meilleure performance.

Une autre difficulté de cette étape tient à la présence du connecteur électrique situé à l'extrémité proximale de la sonde : le diamètre de ce connecteur étant supérieur à celui de la lumière interne du cathéter-guide, il empêche de retirer le cathéter-guide en le faisant simplement glisser vers l'arrière le long de la sonde.

L'étape d'extraction du cathéter-guide impose donc le plus souvent de le découper à son extrémité proximale le long d'une génératrice au moyen d'un outil à lame, dit *slitter*, qui vient fendre l'extrémité proximale du cathéter-guide et le treillis métallique formant l'armature de la gaine creuse. D'une main, le chirurgien tire alors le cathéter-guide vers lui d'un geste continu, en maintenant fermement de l'autre main la sonde et l'outil de découpe qui vient simultanément fendre la gaine au fur et à mesure de son extraction.

On a certes proposé, pour éviter le recours à un outil tranchant, d'utiliser une gaine non armée, simplement pelable ; malheureusement, le prédécoupage de cette gaine sur toute sa longueur entraîne une faiblesse et une moindre rigidité du cathéter-guide, avec un risque de pliure et une moins bonne transmission des efforts lors de sa mise en place. Il a été également proposé, notamment par le EP-A-1 155 710 (ELA Medical) d'équiper la sonde d'un connecteur démontable, ce qui évite d'avoir à découper le cathéter-guide. Cette manière de procéder entraîne cependant une multiplication des étapes de mise en place et d'assemblage des divers éléments, compliquant d'autant l'intervention.

La découpe du cathéter-guide est donc, en pratique, la manière la plus courante de procéder.

L'une des principales difficultés de cette manière d'opérer réside dans le risque d'un déplacement de la sonde au cours de l'extraction du cathéter. Dans cette hypothèse, il est nécessaire de retirer complètement la sonde et recommencer, depuis le début, la procédure d'introduction et de mise en place de celle-ci, ce qui implique un allongement important de l'intervention, notamment avec les sondes ventriculaires gauches qui sont très longues à mettre en place, avec les risques supplémentaires associés à la prolongation d'une intervention déjà très longue.

Des configurations d'outils de découpe ont été par exemple proposées dans les brevets US 4 687 469 (Osypka), 4 997 424 (Little), 6 159 198 (Gardeski), 5 330 460 (Moss) et 7 029 460 (Gardeski). Ces brevets divulguent diverses manières de maintenir la sonde pendant l'opération de découpe du cathéter-guide et de protéger la sonde de tout endommagement par la lame pendant cette opération.

Le US-A-2007/0079511, correspondant au préambule de la revendication 1, décrit un outil de découpe avec un porte-lame de forme aplatie tenu entre deux doigts (le pouce et l'index). Ce porte-lame comporte dans sa région arrière une empreinte pour le pouce, qui est traversée par un couloir courbe situé dans le prolongement d'un élément tubulaire recevant et guidant la sonde au voisinage de la lame pendant la découpe de la gaine du cathéter-guide.

Concrètement, les dispositifs proposés jusqu'à présent présentent tous un certain nombre d'inconvénients.

En particulier le risque, grave, évoqué plus haut de déplacement de la sonde lors de l'opération de découpe est accru par le fait que la sonde n'est pas maintenue dans la région critique de la découpe du cathéter, région où est concentrée la contrainte pendant l'opération. De ce fait, le risque subsiste d'un déplacement relatif de la sonde par rapport au cathéter, avec formation près de la zone de maintien d'une "boucle" se traduisant par un risque élevé de déplacement de l'extrémité opposée (distale) située au site d'implantation, ce déplacement nécessitant une réitération complète de la procédure.

Par ailleurs, dans les outils proposés jusqu'à présent, la préhension de l'outil est assurée par un pincement exercé entre le pouce et l'index, avec un risque d'instabilité pendant la manipulation d'autant plus élevé que la force de découpe est importante, notamment dans le cas de cathéters-guides relativement épais et fortement armés.

En ce qui concerne la préhension de la sonde, celle-ci est également assurée par les doigts, mais à une distance relativement importante de la région où est effectivement réalisée la découpe du cathéter-guide, diminuant ainsi la stabilité de l'ensemble pendant l'opération.

En résumé, les outils proposés jusqu'à présent requièrent toujours un doigté très précis et une grande habitude de la part du chirurgien pour éviter une erreur de manipulation qui serait fatale.

Le but de l'invention est de remédier à l'ensemble de ces difficultés, en proposant un outil de découpe de type *slitter* qui, du point de vue de l'ergonomie, soit supérieur à ce qui a pu être proposé jusqu'à présent et permettre une manipulation simple et sûre par le chirurgien.

L'invention propose à cet effet un outil de découpe du type général décrit dans le US-A-2007/0079511 précité, et comprenant les éléments énoncés dans la partie caractérisante de la revendication 1 pour atteindre les buts exposés ci-dessus.

Diverses caractéristiques subsidiaires avantageuses sont énoncées dans les sous-revendications.

On va maintenant décrire un exemple de mise en oeuvre de l'invention, en référence aux dessins annexés où les mêmes références numériques désignent d'une figure à l'autre des éléments identiques.

Les Figures 1 et 2 sont des vues en élévation, respectivement de face et de dos, de l'outil de découpe de l'invention.

La Figure 3 est une vue perspective, de trois-quarts dessous, de l'outil de découpe des Figures 1 et 2.

La Figure 4 est un détail de la Figure 3, montrant plus précisément les divers éléments constitutifs de la région de découpe.

La Figure 5 est une vue de bout du détail illustré Figure 4.

La Figure 6 illustre schématiquement l'extraction et la découpe du cathéter-guide par le chirurgien au moyen de l'outil de l'invention.

La Figure 7 illustre l'ergonomie de l'outil de l'invention, notamment la manière dont sa forme est adaptée à la morphologie de la main.

On va maintenant décrire un exemple de réalisation de l'outil de l'invention.

Sur les figures, la référence 10 désigne de façon générale l'outil de découpe ou *slitter* selon l'invention, qui est constitué d'un corps porte-lame 12 de forme sensiblement aplatie, comprenant une région de découpe 14 et une région de préhension 16.

Cet outil présente par exemple les dimensions hors-tout typiques suivantes : longueur (plus grande dimension longitudinale de l'outil) 65 à 100 mm, longueur (plus grande dimension transversale) 35 à 40 mm, épaisseur 5 à 6 mm. Ces dimensions ne sont bien entendu données qu'à titre d'exemple, sans aucun caractère limitatif ; on notera seulement que la taille de l'outil doit être telle qu'il puisse être saisi dans le creux de la main du chirurgien, et non plus seulement entre le pouce et l'index seuls, comme cela était le cas des divers outils jusqu'à présent proposés par la technique antérieure.

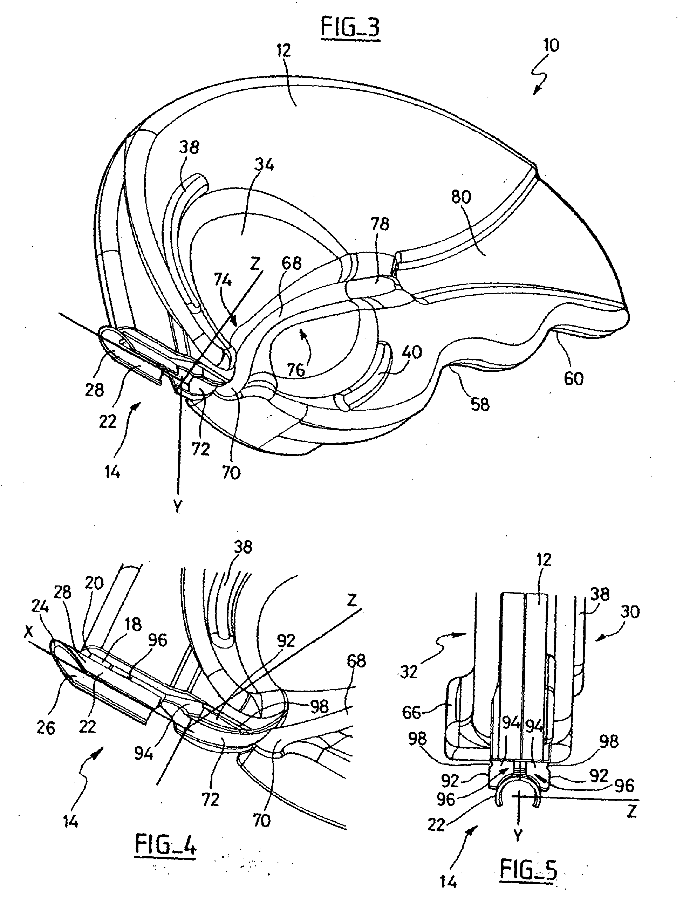

La région de découpe 14 comporte (voir notamment Fig. 4) une lame 18 dont le bord tranchant 20 est tourné vers une direction X, désignée par la suite "direction de découpe", correspondant à la génératrice de l'incision à former dans la gaine du cathéter-guide à extraire. Les autres directions sont la direction latérale Y, perpendiculaire à la direction de découpe X et située dans le plan du corps porte-lame aplati 12, et la direction verticale Z, perpendiculaire au plan du corps porte-lame (ce dernier s'étendant donc dans le plan XY).

Il est également prévu dans la région de découpe 14 un guide tubulaire rectiligne 22 dont la paroi 24 est ouverte vers le bas, c'est-à-dire dans la direction Y. Le guide tubulaire 22 définit un volume intérieur cylindrique 26 dont le diamètre intérieur correspond au diamètre extérieur de la sonde, de manière à loger celle-ci et la protéger de la lame 20. Le cathéter-guide, quant à lui, viendra glisser sur la surface extérieure 28 du guide tubulaire 22. En d'autres termes, le guide tubulaire 22 viendra s'interposer entre la sonde (à protéger) et le cathéter-guide (à découper).

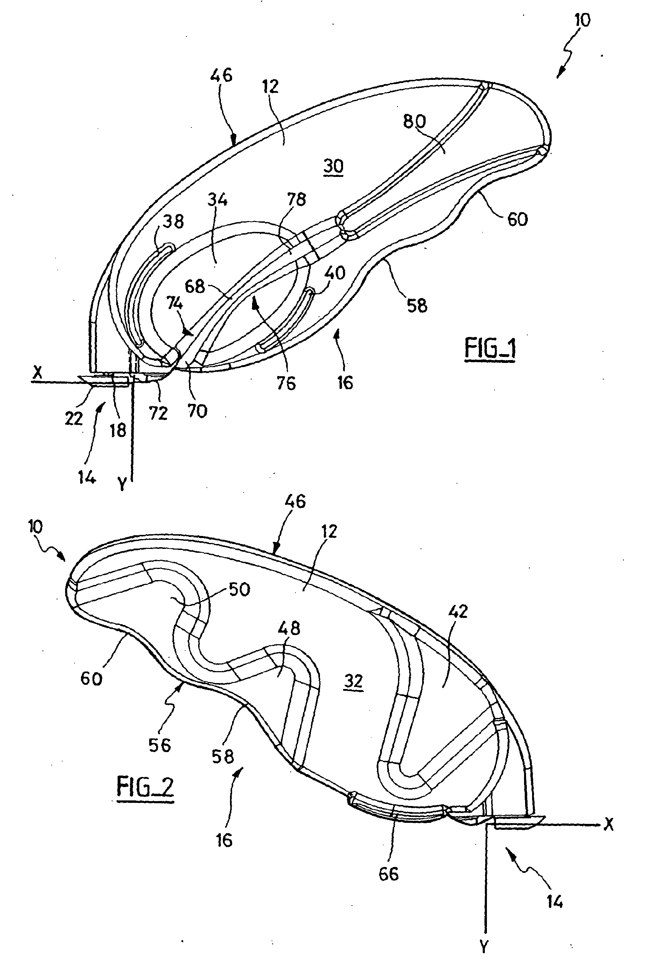

Dans la région de préhension 16, le corps porte-lame 12 présente une première face 30, illustrée Figure 1 et une seconde face, opposée, 32, illustrée Figure 2.

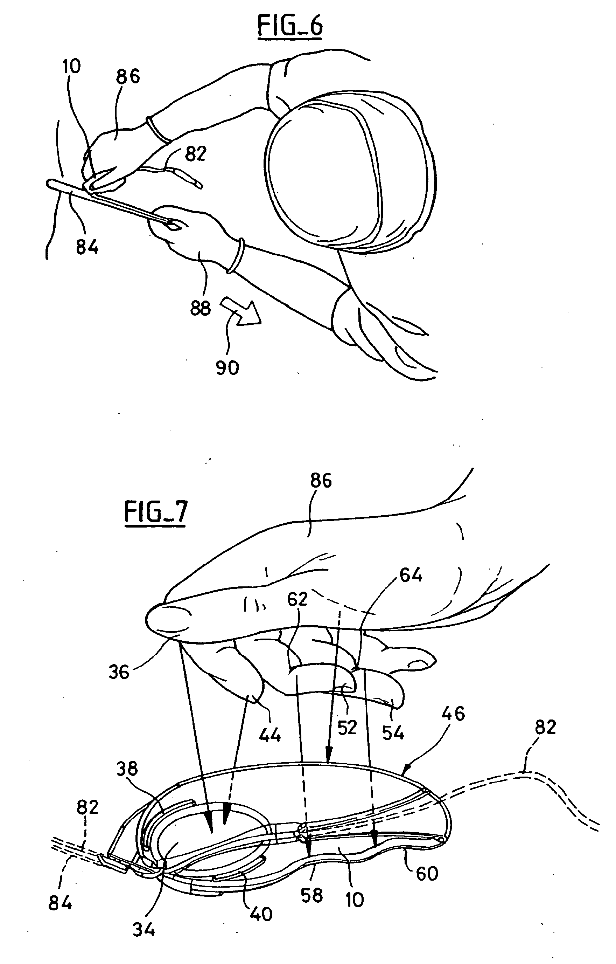

La face 30 comprend une empreinte concave 34 conformée et dimensionnée de manière à recevoir le pouce 36 du chirurgien (voir Fig. 7). Cette empreinte concave 34 est avantageusement pourvue de reliefs de butée opposés 38, 40 permettant d'éviter tout glissement du pouce hors de la cavité pendant l'opération.

Très avantageusement, selon l'invention, la distance minimale entre la lame 18 de la région de découpe 14 et l'empreinte 34 de la région de préhension 16 est réduite à une dimension au plus égale à 20 mm, de préférence au plus égale à 15 mm, de manière à maximiser la stabilité d'ensemble au moment de la découpe.

D'autre part, très avantageusement, la région de l'empreinte 34 est réalisée en un matériau différent de celui du reste du corps porte-outil 12 : le matériau dans la zone 34 est un matériau plus souple, par exemple un matériau élastomère co-moulé avec le reste du corps porte-lame 12, plus rigide (notamment pour un bon assujettissement de la lame). Cette déformabilité supérieure à celle du matériau du reste du corps porte-lame améliore la friction et la conformation du matériau sur la sonde.

La seconde face 32 du corps porte-lame (Fig. 2) comporte une empreinte 42 de réception de l'index 44 (Fig. 7), et son rebord supérieur 46 présente un profil arrondi dimensionné et adapté pour venir dans le creux de la main avec la meilleure tenue et le meilleur confort possibles.

La face 32 comporte également deux empreintes additionnelles concaves 48, 50 pour les phalanges distales respectivement du majeur 52 et de l'annulaire 54. Le profil inférieur 56 est quant à lui conformé avec deux indentations 58, 60 où viennent se placer respectivement les articulations 62, 64 entre phalanges médianes et phalanges distales du majeur et de l'annulaire.

Enfin, il est prévu dans le prolongement de l'empreinte 42, à proximité de la zone de découpe 14, un relief de butée 66 permettant de protéger l'extrémité de l'index du chirurgien pendant l'opération de découpe.

La conformation que l'on vient de décrire permet un excellent maintien de l'outil par le chirurgien, du fait de sa large prise en main et de la manière de le tenir qui permet de résister aux diverses sollicitations, relativement élevées, exercées au niveau de la lame lors de la découpe et de l'extraction du cathéter-guide.

On va maintenant décrire la manière dont l'outil est conformé pour recevoir et maintenir la sonde de la manière la plus efficace pendant cette opération.

L'empreinte concave 34 est traversée à cet effet par une gorge ou couloir de maintien de sonde 68, de part en part dans la plus grande dimension de cette empreinte.

L'une des extrémités 70 du couloir de maintien 68 est située à proximité de la région de découpe 14, et il est prévu à cet endroit, pour assurer une transition avec le guide tubulaire 22, une partie de transition 72 de forme incurvée et arrondie (Fig. 4) permettant de supporter la sonde de façon quasi-continue depuis le guide tubulaire 22, via l'élément 72, jusqu'au couloir de maintien 68.

Le couloir de maintien 68 traversant l'empreinte 34 s'étend dans le plan XY, qui est celui du corps porte-lame, suivant une direction générale formant un angle par rapport à la direction de découpe X, définissant ainsi une première région de courbure 74, prolongée par la forme arrondie de l'élément 72.

Avantageusement, le couloir de maintien 68 présente dans la région de l'empreinte 34 une forme en S, c'est-à-dire que la courbure 74 est prolongée par une contre-courbure 76 en direction de l'extrémité 78 opposée à l'extrémité 70 voisine de la région de découpe 14. Enfin, à l'extérieur de l'empreinte 34, le couloir de maintien 68 est prolongé par un évasement 80, correspondant à une région où la sonde ne présente pas, ou peu, de risques d'être mécaniquement sollicitée. La zone 70 opposée est également légèrement évasée, pour faciliter le placement de la sonde dans le couloir de maintien.

La profondeur (en direction Z) du couloir de maintien 68 dans la région de l'empreinte 34 est, avantageusement, légèrement inférieure au diamètre de la sonde. De la sorte, au centre de l'empreinte 34 dans la zone où l'effort d'appui du pouce sera le plus important, la sonde pourra être directement comprimée par le pouce, assurant ainsi un maintien maximal de celle-ci sur l'outil de découpe.

On notera que la géométrie particulière que l'on vient de décrire procure une immobilisation particulièrement efficace de la sonde, dans la mesure où celle-ci est bloquée dans deux plans perpendiculaires : dans le plan XZ par le guide tubulaire 22, puis dans le plan XY par le couloir de sonde 68. Une telle configuration est favorable à un blocage de la sonde favorisant le maintient et la réduction des degrés de liberté.

L'outil de découpe de l'invention est utilisé de la manière suivante, que l'on va décrire en référence aux Figures 6 et 7.

Après avoir placé la sonde 82 dans le guide tubulaire 22 et dans le couloir 68, le chirurgien positionne la lame de l'outil 10 contre l'extrémité libre du cathéter-guide 84. L'outil 10 est maintenu immobile d'une main 86, avec la sonde fermement assujettie à celui-ci par le pouce de cette main.

De l'autre main 88, le chirurgien extrait le cathéter-guide 84 en le tirant vers l'arrière (flèche 90), jusqu'à extraction complète.

La surface du guide tubulaire 22 comprend avantageusement un matériau antifriction, par exemple un revêtement de métal. Ceci améliore le glissement de la sonde dans cette zone de compression. En effet, lors du geste de découpe, la sonde est fortement comprimée par friction avec le cathéter, et cette compression augmente la friction accumulée juste devant la lame de l'outil de découpe. Si la sonde est entourée d'un matériau antifriction en amont de la zone de découpe, alors la friction dans cette zone peut être réduite, ce qui facilite la découpe, tout particulièrement si le guide tubulaire 22 est nettement proéminent en amont de la lame 18 dans la direction de découpe X.

Pendant l'opération d'extraction, on notera que le cathéter est fendu le long d'une génératrice, de sorte qu'il subsiste le long de l'incision deux lèvres sont le bord peut être dommageable, notamment en raison des fils d'armature coupés du cathéter.

Pour éviter que ces lèvres n'endommagent la sonde pendant l'extraction, il est prévu dans la région de découpe 14, de chaque côté de l'élément 72 de transition du couloir de sonde, des reliefs écarteurs 92 reliés chacun par une rampe 94 à la zone 96 située immédiatement en arrière de la lame 18. Les deux lèvres du cathéter pourront être ainsi écartées l'une de l'autre dans la région où la sonde n'est plus protégée par le guide tubulaire 22. Par ailleurs, des sillons de guidage 98 prolongent la rampe 94 de manière à éviter tout glissement des lèvres du cathéter dans la région critique où il y a lieu de protéger la sonde de tout contact avec ces dernières.

Sur les figures, ces sillons de guidage ont été représentés positionnés en avant du couloir de maintien 68, mais ils peuvent l'être également en arrière de ce couloir, pour assurer un guidage plus long des lèvres découpées du cathéter, tout particulière de part et d'autre de la région où la sonde et le cathéter s'éloignent l'un de l'autre.

L'outil de l'invention présente de nombreux avantages majeurs par rapport aux outils de découpe existants.

Ainsi, en donnant à la sonde une forme sinueuse dans la région de maintien, on réduit très fortement les risques de glissement entre l'outil et la sonde, cette dernière étant ainsi "verrouillée" en place par l'appui du pouce dans l'empreinte correspondante.

D'autre part, la sonde étant guidée sur pratiquement toute sa longueur entre le point de découpe et celui où elle est bloquée dans la cavité, le risque de déplacement est extrêmement faible. De plus, en raison de la très grande proximité entre le contour de l'empreinte recevant le pouce et la lame, le risque de formation d'une boucle, et donc de déplacement de l'extrémité distale de la sonde, est extrêmement réduit.

On notera par ailleurs que l'effort exercé par le pouce sur l'outil, et donc sur la sonde, l'est suivant la direction Z, perpendiculairement au plan XY de l'outil et donc à la direction de découpe X. L'extraction du cathéter - suivant cette direction X - ne produira aucune composante perturbatrice dans la direction perpendiculaire Z et n'affectera donc pas la pression exercée par le chirurgien pour maintenir la sonde.

Enfin, le fait que l'outil soit dimensionné pour être tenu au creux de la main, et non simplement pincé entre le pouce et l'index, procure une ergonomie bien meilleure qui augmente considérablement la stabilité du maintien de la sonde et la précision de la découpe pendant l'extraction du cathéter-guide.

## Revendications

1. Un outil (10) de découpe de la gaine tubulaire d'un cathéter-guide (84) suivant une génératrice de cette gaine en présence d'une sonde (82) logée dans une lumière interne de cette gaine,
cet outil comportant un corps porte-lame (12) de forme sensiblement aplatie apte à être tenu entre les doigts d'un utilisateur, ce corps porte-lame comprenant :
- une région de découpe (14) avec :
**·** une lame (18) présentant un bord tranchant (20) s'étendant sensiblement dans le plan médian du corps porte-lame, ce bord tranchant étant dirigé vers l'extérieur du corps porte-lame suivant une direction de découpe (X) correspondant à ladite génératrice de la gaine à découper, et
**·** un guide tubulaire rectiligne (22), sensiblement parallèle à la direction de découpe, ce guide tubulaire étant apte à recevoir la sonde d'un côté ouvert, opposé à la lame, et étant apte à recevoir le cathéter-guide du côté de la lame,
- une région de préhension (16) prolongeant la zone de découpe en arrière de celle-ci, avec :
**·** sur une première face (30) du corps porte-lame, une zone de réception du pouce de l'utilisateur, comprenant une première empreinte concave (34) formée dans la première face (30) du corps porte-lame et
**·** sur une seconde face (32), opposée, du corps porte-lame, une zone de réception de l'index de l'utilisateur, et
**·** un couloir de maintien de sonde (68) s'étendant dans la région de préhension suivant un parcours comprenant au moins une région courbée (74) traversant de part en part la première empreinte (34) et en prolongement du guide tubulaire et suivant une orientation d'ensemble formant un angle avec ce guide tubulaire,
cet outil étant **caractérisé en ce que** :
- la distance minimale entre la lame (18) et le contour de la première empreinte (34) est au plus égale à 20 mm, et
- le couloir de maintien de sonde (68) s'étend dans la région de préhension suivant un parcours en S comprenant au moins une région courbée (74) et une région contre-courbée (76).

2. L'outil de la revendication 1, dans lequel la distance minimale entre la lame (18) et le contour de la première empreinte (34) est au plus égale à 15 mm.

3. L'outil de la revendication 1, dans lequel le rebord du corps porte-lame (12) présente, dans la région opposée à la lame, un profil (46) conformé à la courbe d'une paume de main.

4. L'outil de la revendication 3, dans lequel le rebord du corps porte-lame (12) présente, dans la région opposée audit profil conformé à la courbe d'une paume de main, un profil (56) avec au moins une indentation (58, 60) de réception des phalanges de la main.

5. L'outil de la revendication 4, dans lequel la seconde face du corps porte-lame comprend au moins une troisième empreinte concave (48, 50) de réception d'une phalange distale de la main.

6. L'outil de la revendication 1, dans lequel la zone de réception de l'index comprend une deuxième empreinte concave (42), formée dans la seconde face (32) du corps porte-lame.

7. L'outil de la revendication 1, dans lequel il est prévu en outre des reliefs (38, 40) de butée du pouce, formés sur le contour de la première empreinte concave (34) de part et d'autre dudit couloir de maintien de sonde (68).

8. L'outil de la revendication 1, dans lequel il est prévu en outre un relief (66) de butée de l'index, formé sur la seconde face (32) du corps porte-lame (12) au voisinage de la zone de découpe (14).

9. L'outil de la revendication 1, dans lequel le couloir de maintien de sonde (68) est évasé en largeur dans sa région d'extrémité (70) s'étendant à proximité du guide tubulaire (22).

10. L'outil de la revendication 1, dans lequel la profondeur du couloir de maintien de sonde (68) est choisie, dans la région centrale de la première empreinte concave (34), inférieure au diamètre nominal de la sonde.

11. L'outil de la revendication 1, dans lequel le matériau du corps porte-lame est un matériau présentant, dans la région de la première empreinte (34), une déformabilité supérieure à celle du matériau du reste du corps porte-lame (12).

12. L'outil de la revendication 1, dans lequel la surface du guide tubulaire (22) comprend un matériau antifriction, notamment un métal.

13. L'outil de la revendication 1, dans lequel la région de découpe (14) comprend en outre, en arrière de la lame (18), des reliefs (92) écarteurs des lèvres du cathéter-guide découpé, ces reliefs écarteurs étant disposés de part et d'autre et en arrière dudit guide tubulaire (22).

14. L'outil de la revendication 13, dans lequel lesdits reliefs écarteurs (92) sont chacun prolongés en arrière par des sillons (98) respectifs de guidage des lèvres du cathéter-guide découpé, ces sillons s'étendant parallèlement à la direction de découpe (X).

15. L'outil de la revendication 14, dans lequel les sillons de guidage (98) sont positionnés en avant, éventuellement en arrière aussi, du couloir de maintien (68).

## Claims

1. A tool (10) for cutting the tubular sheath of a guide catheter (84) along a generatrix of said sheath in the presence of a lead (82) placed in an internal lumen of this sheath, said tool comprising a blade holder body (12) having a substantially flat shape, adapted to be held between the fingers of a user, said blade holder body comprising :
- a cutting region (14) comprising :
- a blade (18) having a sharp edge (20) substantially extending in the median plane of the blade holder body , wherein said sharp edge is directed toward the outside of the blade holder body along a cutting direction (X) corresponding to said generatrix of the sheath to be cut, and
- a straight tubular guide (22) substantially parallel to the cutting direction, said tubular guide being adapted to receive the lead at an open side opposite to the blade and to receive the guide catheter at the blade side,
- a gripping region (16) prolonging the cutting region behind the latter, comprising :
- on a first side (30) of the blade holder body an area for receiving the thumb of the user, comprising a first concave footprint (34) formed in the first side (30) of the blade holder body, and
- on a second, opposite side (32) of the blade holder body an area for receiving the forefinger of the user, and
- a channel (68) for maintaining the lead, extending into the gripping region along a pathway comprising at least one curved area (74) crossing from one side to the other the first footprint (34) and in the continuation of the tubular guide and following an overall orientation presenting an angle with said tubular guide,
wherein said tool is **characterized in that** :
- the minimal distance between the blade (18) and the contour of the first footprint (34) is at most equal 20 mm, and
- the lead maintaining channel (68) extends into the gripping region by following a pathway S comprising at least one curved area (74) and a counter-curved area (76).

2. The tool of claim 1, wherein the minimal distance between the blade (18) and the contour of the first footprint is at most equal to 15 mm.

3. The tool of claim 1, wherein the edge of the blade holder body (12) comprises in the region opposite to the blade a profile (46) conformed to the curvature of the palm of hand.

4. The tool of claim 3, wherein the edge of the blade holder body (12) comprises in a region opposite to said profile conformed to the curvature of a palm of hand a profile (56) having at least one indentation (58,60) for receiving phalanxes of the hand.

5. The tool of claim 4, wherein the second side of the blade holder body comprises at least a third concave footprint (48, 50) for receiving a distal phalanx of the hand.

6. The tool of claim 1, wherein the area for receiving the forefinger comprises a second concave footprint (42) formed in the second side (32) of the blade holder body.

7. The tool of claim 1, wherein stop embossments (38, 40) for a thumb are further provided, that are positioned along the contour of the first concave footprint (34) on both sides of said lead maintaining channel.

8. The tool of claim 1, wherein a stop embossment (66) for the forefinger is further provided, that is positioned on the second side (32) of the blade holler body in the vicinity of the cutting region (14).

9. The tool of claim 1, wherein the lead maintaining channel (68) is laterally flared in its end area extending in the vicinity of the tubular guide (22).

10. The tool of claim 1, wherein the depth of the lead maintaining channel (68) is chosen in the central area of the first concave footprint (34) to be less than the nominal diameter of the lead.

11. The tool of claim 1, wherein the material of the blade holder body is a material having in the area of the first footprint (34) a deformability that is higher than that of the rest of the blade holder body.

12. The tool of claim 1, wherein the surface of the tubular guide (22) comprises an antifriction material, especially a metal.

13. The tool of claim 1, wherein the cutting region (14) further comprises behind the blade (18) embossments (92) acting as retractors of the lips of the cut guide catheter, said retractor embossments being placed at both sides and behind said tubular guide (22).

14. The tool of claim 13, wherein said refractor embossments (92) are each prolonged behind them by respective grooves (98) extending in parallel to the cutting direction (X).

15. The tool of claim 14, wherein the guiding grooves (98) are positioned in front and possibly also behind the maintaining channel.

## Patentansprüche

1. Werkzeug (10) zum Schneiden der röhrenförmigen Umhüllung eines Führungskatheters (84) entlang der Erzeugende genannter Umhüllung in Anwesenheit von einer in einem internen Lumen genannter Umhüllung aufgenommenen Sonde (82), wobei genanntes Werkzeug einen eine flache Form aufweisenden Messerhalterkörper (12) aufweist, die geeignet ist, zwischen den Fingern eines Anwenders gehalten zu sein, wobei genannter Messerhalterkörper
- einen Schnittbereich (14) mit
- einem eine etwa im Mittelplan des Messerhalterkörpers erstreckende Schneide (20) aufweisenden Messer (18), wobei genannte Schneide nach aussen des Messerhalterkörpers entlang einer genannten Erzeugende der zu schneidenden Umhüllung entsprechenden Schnittrichtung (X) gerichtet ist, und
- einer geraden röhrenförmigen, der Schnittrichtung etwa parallelen Führung (22), wobei genannte Führung geeignet ist, die Sonde auf der offenen, gegenüber dem Messer liegenden Seite und den Führungskatheter auf der Seite des Messers aufzunehmen,
- einer Griffzone (16), die den Schnittbereich jenseits letzteres fortsetzt, mit
- auf einer ersten Seite (30) des Messerhalterkörpers einem Bereich zur Aufnahme des Daumens des Anwenders, der eine erste in der ersten Seite (30) des Messerhalterkörpers geformte konkave Aussparung (34), und
- auf einer zweiten gegenüberliegenden Seite (32) des Messerhalterkörpers einem Bereich zur Aufnahme des Zeigefingers des Anwenders, und
- einer die Sonde aufrechterhaltende Rutsche (68), die sich in der Griffzone entlang einer Strecke erstreckt, die wenigstens einen bogenförmigen, die erste Aussparung (34) von einer Seite zur anderen durchdringenden, die röhrenförmige Führung fortsetzenden und einer einen Winkel mit dieser röhrenförmigen Führung bildenden Richtung folgenden Bereich (74) aufweist,
aufweist,
**dadurch gekennzeichnet, dass**
- der Mindestabstand zwischen dem Messer und der Peripherie der ersten Aussparung (34) höchstens 20 mm beträgt, und
- die die Sonde aufrechterhaltende Rutsche (68) sich in der Griffzone entlang einer S-förmigen Strecke erstreckt, die wenigstens einen bogenförmigen Bereich (74) und einen gegenbogenförmigen Bereich (76) aufweist.

2. Das Werkzeug gemäss Anspruch 1, wobei der Mintestabstand zwischen dem Messer (18) und der Peripherie der ersten Aussparung (34) höchstens 15 mm beträgt.

3. Das Werkzeug gemäss Anspruch 1, wobei der Rand des Messerhalterkörpers (12 im dem Messer gegenüberliegenden Bereich ein Profil (46) aufweist, das der Krümmung eines Handtellers entspricht.

4. Das Werkzeug gemäss Anspruch 3, wobei der Rand des Messerhalterkörpers (12) im Bereich, der dem der Krümmung eines Handtellers entsprechenden Profil gegenüberliegt, ein Profil aufweist, das wenigstens eine Vertiefung (58, 60) zur Aufnahme der Phalangen der Hand aufweist.

5. Das Werkzeug gemäss Anspruch 4, wobei die zweite Seite des Messerhalterkörpers wenigstens eine dritte konkave Aussparung (48, 50) zur Aufnahme einer distalen Phalanx der Hand aufweist.

6. Das Werkzeug gemäss Anspruch 1, wobei der Bereich zur Aufnahme des Zeigefingers eine zweite konkave Aussparung (42) aufweist, die in der zweiten Seite (32) des Messerhalterkörpers gebildet ist.

7. Das Werkzeug gemäss Anspruch 1, wobei ausserdem Vorsprünge (38, 40) zum Anschlag des Daumens vorgesehen sind, die auf der Peripherie der ersten konkaven Aussparung (34) beiderseits der die Sonde aufrechterhaltenden Rutsche (68) gebildet sind.

8. Das Werkzeug gemäss Anspruch 1, wobei ausserdem ein Vorsprung (66) zum Anschlag des Zeigefingers vorgesehen ist, der auf der zweiten Seite (32) des Messerhaltekörpers (12) in der Nachbarschaft des Schnittbereichs (14) gebildet ist.

9. Das Werkzeug gemäss Anspruch 1, wobei die die Sonde aufrechterhaltende Rutsche seitwärts in ihrem sich in der Nähe der röhrenförmigen Führung (22) erstreckenden Endbereich (70)erweitert ist.

10. Das Werkzeug gemäss Anspruch 1, wobei die Tiefe der die Sonde aufrechterhaltenden Rutsche (68) im zentralen Bereich der ersten konkaven Aussparung (34) kleiner gewählt ist, als der nominale Durchmesser der Sonde.

11. Das Werkzeug gemäss Anspruch 1, wobei das Material des Messerhalterkörpers ein Material ist, das im Bereich der ersten Aussparung (34) eine Deformierbarkeit aufweist, die grösser ist, als die des Materials des Rests des Messerhalterkörpers (12).

12. Das Werkzeug gemäss Anspruch 1, wobei die Oberfläche der röhrenförmigen Führung (22) ein Lagermaterial, insbesondere ein Lagermetall, besitzt.

13. Das Werkzeug gemäss Anspruch 1, wobei der Schnittbereich (14) hinter dem Messer (18) ausserdem Vorsprünge (92) zum Spreizen der Lippen des geschnitten Führungskatheters aufweist, wobei diese Spreizvorsprünge beiderseits der röhrenförmigen Führung (22) und hinter derselben angeordnet sind.

14. Das Werkzeug gemäss Anspruch 13, wobei jeder genannter Spreizvorsprünge (92) rückwärts durch respektive, zur Führung des geschnitten Führungskatheters geeignete, zu der Schnittrichtung (X) parallele Rillen (98) fortgesetzt sind.

15. Das Werkzeug gemäss Anspruch 14, wobei die Führungsrillen (98) vor, möglicherweise auch hinter der Aufrechterhaltungsrutsche (68) angeordnet sind.
